(19) Europäisches Patentamt
European Patent Office
Office européen des brevets

(11) Publication number: 0 089 216 A2

(12) # EUROPEAN PATENT APPLICATION

(21) Application number: 83301392.3

(22) Date of filing: 14.03.83

(51) Int. Cl.[3]: C 07 D 501/28
C 07 D 501/18, C 07 D 501/34
A 61 K 31/545

(30) Priority: 15.03.82 GB 8207429

(43) Date of publication of application: 21.09.83 Bulletin 83/38

(84) Designated Contracting States: AT BE CH DE FR GB IT LI NL SE

(71) Applicant: GLAXO GROUP LIMITED
Clarges House 6-12 Clarges Street
London W1Y 8DH(GB)

(72) Inventor: Miller, Thomas
37 Moorhall Road
Harefield Middlesex(GB)

(72) Inventor: Robinson, John Malcolm
17 Stowe Crescent
Ruislip Middlesex(GB)

(74) Representative: Holmes, Michael John et al,
Frank B. Dehn & Co. European Patent Attorneys Imperial House 15-19 Kingsway
London, WC2B 6UZ,(GB)

(54) **Cephalosporin compounds.**

(57) Novel cephalosporin compounds are described which are characterised by having a phenoxyacetoxymethyl group at the 3-position. The new compounds are particularly useful intermediates in the synthesis of cephalosporin antibiotics having a 3-methyl group carrying the residue of a nucleophile.

EP 0 089 216 A2

# Cephalosporin Compounds

This invention is concerned with novel compounds which may be used in the preparation of cephalosporin compounds having antibiotic activity.

The cephalosporin compounds in this specification are systematically named with reference to "cepham" after J. Amer. Chem. Soc., 1962, 84, 3400; the term "cephem" refers to the basic cepham structure with one double bond.

Many cephalosporin compounds possessing antibacterial activity are known in the art. In general, these have at the 3-position a substituted methyl group, and are also ordinarily substituted at the 4-position by a carboxy group, and at the 7β-position by an acylamido group. In some instances the compounds may additionally be substituted at other positions, for example at the 2-position (e.g. by one or two methyl groups or a methylene group) and/or at the 7α-position (e.g. by a lower alkyl, alkoxy or alkylthio group).

Cephalosporin compounds of this type in which the 3-substituent is other than an acetoxymethyl group, may be prepared by direct nucleophilic displacement of the acetoxy group of a 3-acetoxymethyl cephalosporin. The latter type of cephalosporin compounds have been considered to be convenient starting materials for such nucleophilic displacement reactions, as the 3-acetoxymethyl substituent is present in cephalosporin C, which is produced by fermentation and is generally the starting material used in the preparation of antibacterially active cephalosporins. However, the acetoxy group is not a good leaving group so that the displacement reaction may be unsuccessful with some nucleophiles and in other cases low yields may be obtained.

An alternative starting material which is produced by fermentation and may be used for preparing 3-substituted methyl cephalosporins, is desacetyl cephalosporin C, the 3-hydroxymethyl analogue of cephalosporin C. However, with this starting material it is necessary to introduce, at some stage in the reaction sequence, a readily displaceable substituent on the 3-methyl group (for example by acylation of the hydroxyl group) in order to permit the introduction of the desired substituent.

We have now found that the phenoxyacetoxy group is an effective and particularly useful leaving group in such nucleophilic displacement reactions.

No cephalosporin compounds having a 3-phenoxyacetoxymethyl group have previously been specifically described.

A number of acyloxy groups have previously been described as possible displaceable substituents for nucleophilic displacement reactions at the 3-position of cephalosporins. Thus, British Patent Specification No. 1241657 discloses cephalosporin compounds having a group $-CH_2X$ at the 3-position, which compounds are capable of reaction with a range of nucleophiles to effect ready nucleophilic displacement. The group X is defined as the residue of an acid HX which acid has a pKa value of not more than 4, and examples of such acids include acetic acid derivatives having at least one electron-withdrawing substituent on the α-carbon atom. There is no mention of phenoxyacetic acid in this context. It is stated in British Patent Specification No. 1241657 that it is generally necessary to protect the 4-carboxyl group of the cephalosporin in which X is an acyloxy group in order to prevent lactonisation.

British Patent Specificaion No. 1141293 describes a process for the preparation of cephalosporin antibiotic

compounds having an acyloxymethyl group at the 3-position by aralkylating the 4-carboxyl group of a 3-hydroxymethyl cephalosporin, acylating the 3-hydroxymethyl group of the protected compound, and subsequently removing the aralkyl group from the 4-carboxylic group. Protection of the 4-carboxyl group is effected in order to prevent lactonisation in the subsequent acylation. The acyloxy group of the cephalosporin products may be any carboxylic acyloxy group and it is stated that the acyl moiety at the 3-position may be defined as for the acyl group in the 7-side chain. A lengthy list of suitable 7-acyl groups includes phenoxyacetyl, but compounds having a 3-phenoxyacetoxymethyl group are not exemplified. There is no reference to use of the phenoxyacetoxy group as a leaving group.

As indicated above, we have now found that the phenoxyacetoxy group is a particularly useful leaving group for nucleophilic displacement reactions at the 3-position of cephalosporins. In particular, we have found that during the preparation of 3-phenoxyacetoxymethyl cephalosporin compounds or during a subsequent nucleophilic displacement reaction with them, it is not necessary to protect the 4-carboxyl group in order to prevent lactonisation. Thus, we have found the 3-phenoxyacetoxy group to be a readily displaceable and effective leaving group which, under normal reaction conditions, does not enter into lactone formation to any significant extent with a free carboxyl group at the 4-position of the cephalosporin. The 3-phenoxyacetoxymethyl cephalosporin compounds can therefore be prepared in high yield and can also be converted into 3-(substituted methyl)-cephalosporins in high yield even when the 4-carboxyl group is not protected. The use of the novel intermediates therefore leads to increased economy and efficiency in an overall process for making cephalosporin antibiotics, by the avoidance of steps for protecting and deprotecting the 4-carboxyl group.

Thus, cephalosporin compounds substituted at

the 3-position by a phenoxyacetoxymethyl group are novel compounds according to the invention which are particularly useful intermediates in the synthesis of cephalosporin antibiotics having a 3-methyl group carrying the residue of a nucleophile.

Thus, one aspect of the invention provides compounds of general formula

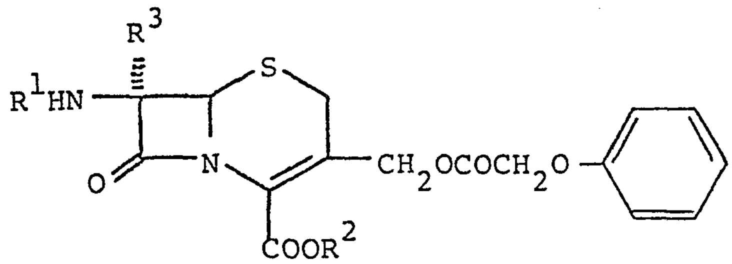

(I)

wherein $R^1$ represents a hydrogen atom or an acyl group; $R^2$ represents a hydrogen atom or a carboxyl blocking group (e.g. the ester forming residue of an alcohol, phenol, silanol or stannanol, the residue preferably being one which may readily be split off); and $R^3$ represents a lower alkyl, alkylthio or alkoxy group (e.g. a methoxy group) or, preferably, a hydrogen atom, and, where appropriate, salts thereof with acids or bases (e.g. alkali metal salts such as sodium or potassium salts, alkaline earth metal salts such as calcium salts, ammonium and organic amine salts, and salts with inorganic or organic acids, such as hydrochloric, sulphuric, hydrobromic, phosphoric, acetic, lactic, maleic, fumaric, succinic, citric, tartaric, p-toluenesulphonic or methanesulphonic acid).

In the compounds of general formula (I), $R^2$ is preferably a hydrogen atom. When $R^2$ represents a carboxyl blocking group, however, this may conveniently be selected from conventional carboxyl blocking groups, for example those described in "Protective Groups in Organic Chemistry" Ed. J.F.W. McOmie (Plenum Press 1973).

When $R^1$ represents an acyl group, this may for example, be selected from the wide range of side-chain acyl groups known in the β-lactam art. It will be

appreciated that where the acylamido group carries substituents such as amino, hydroxy, carboxyl or mercapto groups, these may be protected as appropriate by any conventional protecting group, for example as described in the above mentioned "Protective Groups in Organic Chemistry".

Thus, for example, amino groups may be protected by substitution with any of the following groups: formyl; optionally substituted lower alkanoyl such as acetyl or chloroacetyl; aryl lower alkanoyl such as phenylacetyl; aryloxy lower alkanoyl such as phenoxyacetyl; aroyl such as benzoyl; optionally substituted lower alkoxycarbonyl such as n-butoxycarbonyl, isobutoxycarbonyl, t-butoxycarbonyl; or 2,2,2-trichloroethoxycarbonyl; aryl lower alkoxycarbonyl such as benzyloxycarbonyl or diphenylmethoxycarbonyl; cycloalkoxycarbonyl such as 1-adamantyloxycarbonyl; lower alkylsulphonyl such as methanesulphonyl; arylsulphonyl such as p-toluenesulphonyl; sulphinyl for example o- or p-nitro-phenylsulphinyl; arylmethyl, for example, triphenylmethyl; ylidene groups formed by reaction with aldehydes and ketones which form Schiffs bases, for example benzaldehyde, salicylaldehyde or acetoacetic ester; or the nitrogen atom of the protected amino group may form part of a heterocyclic ring such as phthalimido, or a dihydropyridine ring.

Examples of hydroxyl and mercapto protecting groups include the above mentioned amino protecting carboxylic and sulphonic acyl groups; lower alkyl groups such as t-butyl; aralkyl groups such as benzyl, diphenylmethyl or triphenylmethyl; and silyl groups such as trimethylsilyl.

Carboxyl groups may be protected by formation of an ester, for example with an ester forming residue as mentioned above for $R^2$. Examples of carboxyl blocking groups thus include unsubstituted or substituted alkyl groups e.g. t-butyl and 2,2,2-trichloroethyl; unsubstituted or substituted aralkyl groups e.g. p-methoxybenzyl, p-nitrobenzyl and diphenylmethyl; and silyl groups such as trimethylsilyl.

Where desired, protecting groups may be removed from the cephalosporin compound by methods well known in the art, for example by hydrolytic, reductive or acid-induced cleavage as appropriate. However, as some of these methods may also remove the 3-phenoxyacetyl group, it may be preferable not to remove any protecting groups until after displacement of the phenoxyacetoxy substituent.

Specific acyl groups which may be present when $R^1$ represents an acyl group may, for example, contain up to 40 carbon atoms, and are illustrated in the following list, which is not intended to be exhaustive:-

(i) $R^aC_nH_{2n}CO-$ where $R^a$ is an optionally substituted aryl (carbocyclic or heterocyclic), cycloalkyl, cycloalkadienyl or a non-aromatic or mesoionic heterocyclic group and n is 0 or an integer from 1 - 4. Examples of this group include phenylacetyl, thien-2-and -3-ylacetyl, fur-2- and -3-ylacetyl, 3-and 4-isoxazolylacetyl, pyridylacetyl, tetrazolylacetyl, thiazolylacetyl, thiadiazolylacetyl, cyclohexadienylacetyl or a sydnoneacetyl group, any of which may be optionally substituted e.g. by a halogen atom or a nitro, hydroxy, amino, lower alkyl, lower alkoxy or lower acyloxy group. Where n is other than 0, especially when n is 1, the α-carbon atom of the acyl group may be substituted by, for example, a hydroxy, blocked hydroxy (e.g. lower alkanoyloxy such as acetoxy) amino, protected amino, carboxy, blocked carboxy, sulphonate, hydroxyimino, acyloxyimino (e.g. lower alkanoyloxyimino such as acetoxyimino or halo substituted lower alkanoyloxyimino, such as mono- or dichloroacetoxyimino) or etherified oxyimino (e.g. lower alkoxyimino such as methoxyimino or t-butoxyimino, substituted lower alkoxyimino such as carboxymethoxyimino,

2-carboxyprop-2-oxyimino, lower cycloalkoxyimino or substituted lower cycloalkoxyimino such as 1-carboxycyclobut-1-oxyimino or aryloxyimino such as phenoxyimino) group. Examples of α-substituted acyl groups of this type include 2-(fur-2-yl)-2-methoxyiminoacetyl, 2-(2-aminothiazol-4-yl)-2-methoxyiminoacetyl and 2-(2-aminothiazol-4-yl)-2-(2-carboxyprop-2-oxyimino)acetyl;

(ii) $C_nH_{2n+1}CO$ where $\underline{n}$ is 0 or an integer from 1 to 7. The alkyl group may be straight or branched and may be interrupted by an oxygen or sulphur atom and/or substituted by e.g. a halogen atom, a cyano group, a carboxy or blocked carboxy group, an alkoxycarbonyl group, a hydroxy or blocked hydroxy group or a carboxycarbonyl group (CO.COOH) in which the carboxy portion may be optionally blocked. Examples of such groups include formyl, cyanoacetyl, hexanoyl, heptanoyl, octanoyl, butylthioacetyl and carboxybutanoyl;

(iii)

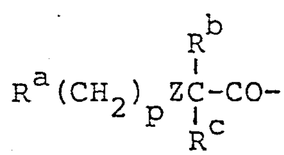

where $R^a$ has the meaning defined under (i), p is 0 or 1, $R^b$ and $R^c$ which may be the same or different each represent hydrogen, phenyl, benzyl, phenethyl, or lower alkyl and Z is an oxygen or sulphur atom. Examples of this group include phenoxyacetyl and pyridylthioacetyl; and

(iv)

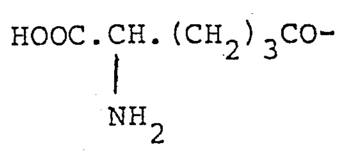

or an N-protected and/or carboxyl-blocked derivative thereof, where the amino protecting and/or carboxyl blocking group is as hereinbefore defined.

The term "lower", as used herein, indicates that the group concerned desirably contains 1-6, preferably 1-4 carbon atoms in the alkyl moiety. Aryl and aroyl groups conveniently contain 6-18 carbon atoms in the aryl moiety.

Compounds according to the invention may be prepared by the following process, which process constitutes a further feature of the invention:-

by phenoxyacetylating a cephalosporin compound having a hydroxymethyl group in the 3-position, more especially a compound of formula

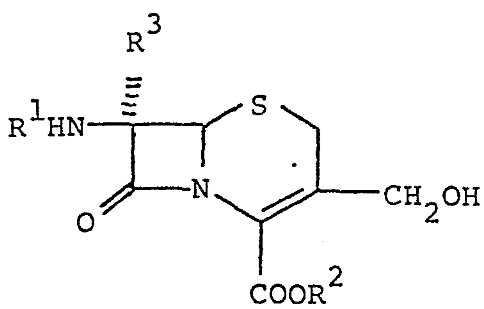

(II)

(wherein $R^1$, $R^2$ and $R^3$ are as hereinbefore defined) or, where appropriate, a salt thereof; whereafter, if necessary and/or desired in each instance, any of the following reactions in any appropriate sequence, may be carried out:-

(i) N-deacylation of a compound wherein $R^1$ is an acylamido group to form a 7-amino compound;

(ii) N-acylation of a compound wherein $R^1$ is hydrogen to form a desired acylamido group $R^1$;

(iii) conversion of a carboxyl group into a salt or ester function;

(iv) removal of any carboxyl blocking group, O-protecting, S-protecting and/or N-protecting groups; and

(v) conversion of a basic group into a salt function.

The phenoxyacetylation step of the process may be effected by any convenient method, for example by reacting the compound of formula (II) with an appropriate acylating agent. The acylating agent may conveniently be a reactive derivative of phenoxyacetic acid, such as a phenoxyacetyl halide e.g. phenoxyacetyl chloride or bromide, or an anhydride or activated ester of phenoxyacetic acid. Alternatively phenoxyacetic acid itself may be used as the acylating agent, in which case a condensing agent is desirably present, for example a carbodiimide such as N,N'-dicyclohexylcarbodiimide or N-ethyl-N'-$\gamma$-dimethylaminopropylcarbodiimide.

The phenoxyacetylation is conveniently effected at a temperature of from -50° to +50°C, preferably 0° to +30°C.

The reaction is advantageously carried out in an inert organic solvent which is preferably anhydrous, and, when the acylating agent is an acyl halide, desirably in the presence of an acid binding agent. Examples of solvents which may be used include amides, such as dimethylacetamide, ethers such as tetrahydrofuran, ketones such as acetone, halogenated hydrocarbons such as methylene chloride and nitriles such as acetonitrile. A preferred solvent is dimethylacetamide. Examples of acid binding agents include tertiary amines e.g. triethylamine or dimethylaniline, inorganic bases e.g. calcium carbonate or sodium bicarbonate, and oxiranes such as lower 1,2-alkylene oxides e.g. ethylene oxide or propylene oxide, which bind hydrogen halide liberated in the acylation reaction.

It will be appreciated that where the phenoxyacetylation reaction is carried out using a compound of formula (II) wherein $R^1$ represents hydrogen, the resulting compound of formula (I) will possess a 7-phenoxyacetamido group as well as a 3-phenoxyacetoxymethyl group. Likewise, if $R^1$ in formula (II) is an acylamido group containing free amino or hydroxyl groups these will become phenoxyacetylated during the reaction. The amount of the phenoxyacetylating agent to be used will therefore depend on how many such reactive groups are present in the starting material

As indicated above, when introducing a readily displaceable substituent at the 3-position it has hitherto generally been necessary to protect the 4-carboxyl group of a 3-hydroxymethyl cephalosporin during acylation in order to prevent formation of a lactone, but we have surprisingly found, that the phenoxyacetylation process of the invention proceeds without lactonisation, even when there is a free carboxyl group at the 4-position of the starting cephalosporin.

The N-deacylation reaction (i) above, may be effected in conventional manner, for example as described in U.K. Patent Specifications Nos. 1 041 985 and 1 391 437.

Likewise the N-acylation reaction (ii) above may be carried out by conventional means, for example as described in U.K. Patent Specification No. 1399086.

Reactions (iii) - (v) above may also be effected by methods known in the art, for example, as described in U.K. Patent Specifications Nos. 1 399 086, 1 342 241 and 2 018 764A.

Starting compounds of formula (II) are known compounds and may, for example, be obtained by N-acylation of 7-amino-3-hydroxymethylceph-3-em-4-carboxylic acid in conventional manner, e.g. as described in British Patent Specification No. 1,399,086.

The novel compounds according to the invention may be used in the preparation of cephalosporin compounds having a wide variety of 3-(substituted methyl) groups. Thus, the compounds may be subjected to nucleophilic displacement reactions, such as with nitrogen, sulphur and oxygen nucleophiles. These reactions may be effected analogously to methods well known in the art e.g. as described in British Patent Specifications Nos. 1,399,086, 2,025,398 and 2,027,691.

As indicated above, the basic starting materials in an overall process for the preparation of antibacterially useful cephalosporin compounds carrying a 3-substituted methyl group are, in general, cephalosporin C, i.e.(6R,7R)-7-[D-5-amino-5-carboxypentanamido]-3-(acetoxymethyl)ceph-3-em-4-carboxylic acid and salts thereof, such as its sodium or potassium salt, or desacetyl cephalosporin C, that is the 3-hydroxymethyl analogue of cephalosporin C, and its salts. Cephalosporin C may be converted into desacetyl cephalosporin C, for example, by the processes described in our U.K. Patent Specifications Nos. 1 474 519 and 1 531 212. The compounds according to the invention may be used in such overall processes, for example, by phenoxyacetylating desacetyl cephalosporin C, or, more preferably, an N-protected derivative thereof, to form a compound according to the invention in which $R^1$ is an N-protected aminoadipoyl group, whereafter the 3-position group of the desired final product may be introduced by nucleophilic displacement. Where appropriate, the N-protected aminoadipoyl group may then be replaced by the 7-acyl substituent desired in the final cephalosporin product. Thus, preferred compounds of formula (I) for use as intermediates include those derived from desacetyl cephalosporin C, and have the formula

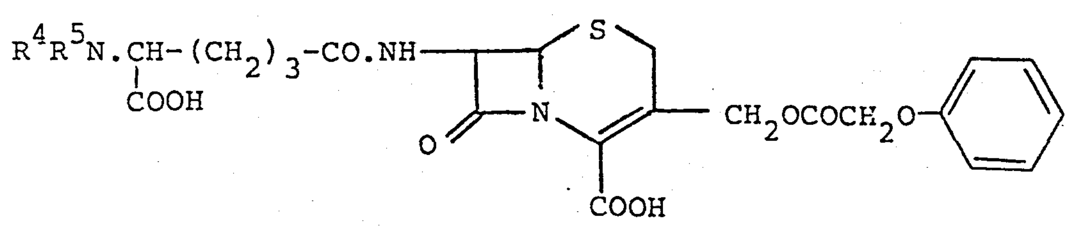

(wherein $R^4$ is hydrogen and $R^5$ is a monovalent amino protecting group e.g. as described above, or $R^4$ and $R^5$ together form a divalent amino protecting group e.g. as described above) and salts thereof. Preferably $R^4$ is hydrogen and $R^5$ represents an N-benzoyl group.

In addition to their principal use as intermediates for the preparation of cephalosporin antibiotics compounds according to the invention themselves exhibit antibacterial activity against a range of gram-positive and gram-negative organisms, such as <u>Staphylococcus</u>

*aureus*, *Escherichia coli*, *Providence* species and *Haemophilus influenzae*. Antibiotic compounds of formula (I) may thus be used to treat a variety of bacterial infections.

Such compounds will generally possess a 7-acylamido group present in known antibacterially active cephalosporins. Thus, for example, in antibiotic compounds of formula (I) $R^1$ may be a group of formula:

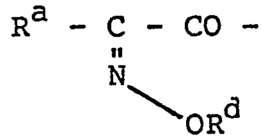

(wherein $R^a$ has the above meaning, preferably an optionally substituted phenyl, thienyl, furyl, isoxazolyl, pyridyl, tetrazolyl, thiazolyl, thiadiazolyl, cyclohexadienyl or sydnone group; $R^d$ is a hydrogen atom, an optionally substituted lower alkyl or cycloalkyl group or an acyl group; and the group $-OR^d$ is in the *syn*-configuration with respect to the carbonyl group).

The antibiotic compounds of the invention may be formulated for administration in any convenient way, by analogy with other antibiotics and the invention therefore includes within its scope pharmaceutical compositions comprising an antibiotic compound in accordance with the invention adapted for use in human or veterinary medicine. Such compositions may be presented for use in conventional manner with the aid of any necessary pharmaceutical carriers or excipients.

The antibiotic compounds according to the invention may be formulated for injection and may be presented in unit dose form in ampoules, or in multi-dose containers, if necessary with an added preservative. The compositions may also take such forms as suspensions, solutions, or emulsions in oily or aqueous vehicles and may contain formulatory agents such as suspending, stabilising and/or dispersing agents. Alternatively the active ingredient may be in powder form for reconstitution

with a suitable vehicle, e.g. sterile, pyrogen-free water, before use.

The antibiotic compounds may also be formulated as suppositories, e.g. containing conventional suppository bases such as cocoa butter or other glycerides.

Compositions for veterinary medicine may, for example, be formulated as intramammary preparations in either long acting or quick-release bases.

The compositions may contain from 0.1% upwards, e.g. 0.1-99%, of the active material, depending on the method of administration. When the compositions comprise dosage units, each unit will preferably contain 100-3000mg, e.g. 200 to 2000mg, of the active ingredient. The dosage as employed for adult human treatment will preferably range from 500 to 12000 mg, e.g. 1500 to 9000mg, per day, depending on the route and frequency of administration. For example, in adult human treatment 1000 to 4000 mg per day administered intravenously or intramuscularly will normally suffice.

The antibiotic compounds according to the invention may be administered in combination with other therapeutic agents such as antibiotics, for example penicillins or other cephalosporins.

The following Examples serve to illustrate the invention. All temperatures are in $^{o}C$.

The proton magnetic resonance (p.m.r.) spectrum was determined at 100 MHz. The integrals are in agreement with the assignments; coupling constants, J, are in Hz, the signs not being determined; s=singlet, d=doublet, dd=double doublet, t=triplet, m=multiplet, and ABq=AB quartet.TFA is trifluoroacetic acid.

Amberlite LA2 is a weakly basic high molecular weight secondary amine sold by Rohm and Haas, Philadelphia U.S.A.

EXAMPLE 1

(6R,7R)-7-[D-5-benzamido-5-carboxypentanamido]-3-(phenoxyacetoxymethyl)ceph-3-em-4-carboxylic acid, monosodium salt

To potassium (6R,7R)-7-(D-5-amino-5-carboxypentanamido)-3-(hydroxymethyl)ceph-3-em-4-carboxylate (8.2g) in N,N-dimethylacetamide (30ml) at $-5^{o}$ was added benzoyl chloride (2.8ml) dropwise with stirring. Stirring was continued at room temperature for one hour when, after cooling to $0^{o}$, phenoxyacetyl chloride (3.3ml) was introduced dropwise. The mixture was stirred for one hour at ambient temperature before methanol (2.5ml) was added. After 15 minutes stirring the mixture was partitioned between ethyl acetate (80ml) and water (40ml) and the organic layer was evaporated to a foam (14.6g) which was taken up in acetone (40ml). A solution of anhydrous sodium acetate (1.64g) in methanol (20ml) was added and, after ageing in the cold, the crystalline solid which deposited was collected by vacuum filtration, washed with methanol: acetone (1:2) and with acetone and dried at room temperature *in vacuo* to give the *title compound* (7.0g). τ (DMSO - $d_6$); 1.30 (d, J 8Hz, 7-amido-NH); 1.54(d, J 7Hz, benzamido - NH); 2.0 to 3.2 (benzamido- and phenoxy-); 4.50 (dd, J 8, 4Hz, $C_7H$); 5.09 (d, J 4Hz, $C_6H$); 4.86 and 5.10 (ABq, J 12 Hz, $C_3$-$CH_2$-); 5.24 (phenoxyacetate-$CH_2$); and 7.6 to 8.0 (-$(CH_2)_2$-$C\underline{H}_2$-CO).

EXAMPLE 2

(6R,7R)-7-[D-5-phenoxyacetamido-5-carboxypentanamido]-3-(phenoxyacetoxymethyl)ceph-3-em-4-carboxylic acid, dipotassium salt

Phenoxyacetyl chloride (4.8 ml) was added over 20 minutes to a stirred mixture of potassium (6R,7R)-7-(D-5-amino-5-carboxypentanamido)-3-(hydroxymethyl)ceph-3-em-4-carboxylate (6.5 g) and N,N-dimethylacetamide (24 ml) with ice cooling. The reaction mixture was stirred for 1 hour at 0°, propylene oxide (1.4 ml) being added after 0.5 hours. After 2 hours at ambient temperature the mixture was treated with a solution of potassium carbonate (2.2 g) in water (10 ml) and added over 5 minutes to stirred acetone (650 ml). The resulting suspension was aged in the cold and the supernatant liquor decanted off. The precipitate was slurried with acetone (200 ml), the solid collected by filtration and washed with acetone and dried *in vacuo* to give the *title compound* (10.5 g), microanalysis indicated the presence of KCl; found: C, 43.59; H, 3.82; N, 5.06; K, 15.5; Cl, 6.4%, $C_{30}H_{29}N_3SO_{11}K_2$ + 1.5 moles of KCl requires C, 43.43; H, 3.52; N, 5.06; K, 16.5; Cl, 6.4%; $\tau(D_2O/NaHCO_3)$, 2.5-3.2 (2 x phenyl), 4.42 (d, J 5 Hz, $C_7$-H), 5.06 (d, J 5Hz, $C_6$-H), 4.98 and 5.46 (ABq, J 12Hz, $C_3$-$C\underline{H}_2$-), 5.72 (-C$\underline{H}$-NH), 6.78 and 7.11 (ABq, J 18 Hz, 2H : $C_2$-H), 7.72 (-$C\underline{H}_2$CONH-), 8.0-8.6 (4H : CH-$\underline{CH}_2$-$\underline{CH}_2$).

EXAMPLE 3

(6R,7R)-7-[D-5-phenoxyacetamido-5-carboxypentanamido]-3-(phenoxyacetoxymethyl)ceph-3-em-4-carboxylic acid, monosodium salt

Phenoxyacetyl chloride (4.15 ml) was added dropwise over 20 minutes to a stirred mixture of potassium

(6R,7R)-7-(D-5-amino-5-carboxypentanamido)-3-(hydroxymethyl) ceph-3-em-4-carboxylate (4.2 g) and N,N-dimethylacetamide (15 ml) cooled to -10°. The mixture was stirred for 25 minutes, methanol (3 ml) added, and the mixture partitioned between water (20 ml) and methylene chloride (40 ml). The organic layer was separated, washed with water (3 x 20 ml), dried and evaporated to an oil. The oil was dissolved in methanol (25 ml) and a solution of anhydrous sodium acetate (0.82 g) in methanol (10 ml) added and, after aging in the cold, the deposited solid was collected by filtration, washed with methanol and dried _in vacuo_ to give the _title compound_ (4.2 g), λmax's (pH 6 phosphate at 338 (ε 1130) and 268 nm (ε 8825) and λinf's at 263.5 (ε 8625) and 273.5 nm (ε 7300); τ(DMSO $d_6$), 1.38 (d, J 9Hz, $C_7$-NH), 1.96 (d, J 8Hz, phenoxyacetamido-NH), 2.6-3.2 (2 x phenyl), 4.8-5.4 ($C_3$-C$\underline{H}_2$-), 5.27 (phenoxyacetyl-$CH_2$), 5.53 (phenoxyacetamido-$CH_2$), 5.82 (CH-NH), 7.82 (-C$\underline{H}_2$CONH-), 8.0-8.5 (4H: CH-C$\underline{H}_2$-C$\underline{H}_2$-). Water content by Karl Fischer method 1.0%; sodium content 2.9%, $C_{30}H_{29}N_3O_{11}SNa$ + 1% water requires 3.4%.

EXAMPLE 4

(6R,7R)-7-amino-3-(phenoxyacetoxymethyl)ceph-3-em-4-carboxylic acid

To a suspension of (6R,7R)-7-[D-5-benzamido-5-carboxypentanamido]-3-(phenoxyacetoxymethyl)ceph-3-em-4-carboxylic acid, monosodium salt (2.85 g) in methylene chloride (24 ml) containing dimethylanaline (3.42 ml) was added trimethylchlorosilane (3.44 ml), and the mixture stirred at ambient temperature for 5 minutes. The solution obtained was cooled to -40° and phosphorus pentachloride (2.79 g) added and the mixture stirred at -30° for 1 hour. A mixture of butane-1,3-diol (3.78 ml) and methylene chloride (15 ml) previously cooled to 0°, was added to the reaction

mixture. The mixture was extracted with water (15 ml) and the extract washed with methylene chloride (5 ml). The aqueous layer was cooled to 5°, adjusted to pH 3.6 with concentrated aqueous ammonium hydroxide and the resulting suspension treated with acetone (30 ml) over 10 min. The solid was filtered and the filter bed washed with water and acetone to give the <u>title compound</u> (2.5 g); νmax (Nujol) 1805 $cm^{-1}$ (β-lactam); τ(TFA), 2.65-3.15 (phenyl), 4.65 (s, $C_6$ and $C_7$-H), 4.42 and 4.73 (ABq J 14 Hz, $C_3$-$C\underline{H}_2$-), 5.15 (s, $PhOC\underline{H}_2$-) and 6.4 (s, 2H : $C_2$-H). Water content by Karl Fischer method 2.0%; Found : C, 51.3; H, 4.5; N, 7.9%, $C_{16}H_{16}O_6SN_2$ + 2.0% water requires: C, 51.6; H, 4.5; N, 7.55%.

## EXAMPLE 5

<u>(6R,7R)-7-[D-5-(n-butoxycarbonylamido)-5-carboxypentanamido]-3-(phenoxyacetoxymethyl)ceph-3-em-4-carboxylic acid, monosodium salt</u>

Potassium (6R,7R)-7-(D-5-amino-5-carboxypentamido)-3-(hydroxymethyl)-ceph-3-em-4-carboxylate (40 g) was added to a stirred solution of sodium bicarbonate (17.2 g) in water (200 ml). To the solution obtained was added n-butyl chloroformate (12.4 ml) and the mixture stirred at ambient temperature for 1.5 hours. The reaction mixture was cooled to 5° and extracted with a mixture of ethyl acetate : acetone (2:1, 300 ml) at pH 2.0 using phosphoric acid. The organic extract was treated with ammonium hydroxide (.880, 7.52 ml) and the suspension cooled to 0°, filtered, the filter bed washed with ethyl acetate:acetone (2:1) and acetone, then dried <u>in vacuo</u> at 45° to give (6R,7R)-7-[D-5-(n-butoxycarbonylamido)-5-carboxypentanamido]-3-(hydroxymethyl)ceph-3-em-4-carboxylic acid, monoammonium salt (34.7 g) as a solid. This solid (14.72 g) was added to a stirred mixture of acetyl chloride (2.13

ml) and methanol (1.2 ml) in N,N-dimethylacetamide (45 ml) at 0°. Phenoxyacetyl chloride (9.9 ml) was added over 3 min keeping the temperature of the mixture below 5°. The mixture was stirred for 0.5 hours allowing it to warm to ambient temperature, methanol (10 ml) was added and the mixture stirred for 5 minutes. A mixture of ethyl acetate (120 ml) and half saturated aqueous sodium chloride solution (120 ml) was added, the upper organic layer separated, and washed with half saturated aqueous sodium chloride (3 x 60 ml), and evaporated to an oil. The oil was taken up in acetone (60 ml) and a solution of anhydrous sodium acetate (2.46 g) in methanol (30 ml) added and, after aging in the cold, the solid which deposited was collected by filtration and washed with acetone:methanol (2:1) and acetone and dried <u>in vacuo</u> to give the <u>title compound</u> (13.0 g) λmax's (pH 6 phosphate) at 337.5 (ε 945) and 262 nm (ε 7810) and λinf's at 265.5 (ε 7680) and 273.5 nm (ε 6300); τ($D_2O/NaHCO_3$), 2.5-3.1 (phenyl), 4.39 (d, J 5 Hz, $C_7$-H), 4.9 (d J 5 Hz, $C_6$-H), 4.8-5.4 ($PhOC\underline{H}_2$- and $C_3$-$C\underline{H}_2$), 5.92 ($C\underline{H}$-NH- and -$CO_2C\underline{H}_2$-), 6.57 and 6.9 (ABq J 18 Hz, 2H:$C_2$-H), 7.63 (-$C\underline{H}_2$-CONH-), 8.2-8.8 (4H: CH-$C\underline{H}_2$-$C\underline{H}_2$- and 4H: -$C\underline{H}_2$-$C\underline{H}_2$-$CH_3$) and 9.14 (t, J 7Hz, -$CH_3$).

## EXAMPLE 6

**(6R,7R-7-[D-5-isobutoxycarbonylamido-5-carboxypentanamido]-3-(phenoxyacetoxymethyl)ceph-3-em-4-carboxylic acid, monosodium salt**

Following a similar procedure to that described in Example 5, but using isobutylchloroformate (8.2 ml), potassium (6R,7R)-7-(D-5-amino-5-carboxypentanamido)-3-(hydroxymethyl)ceph-3-em-4-carboxylate (20 g) was converted into (6R,7R)-7-[D-5-isobutoxycarbonylamido-5-carboxypentanamido]-3-(hydroxymethyl)ceph-3-em-4-carboxylic acid, monoammonium salt (11.8 g)

as a solid. Using phenoxyacetyl chloride (6.6 ml) this solid (10.6 g) was converted into the <u>title compound</u> (8.1 g); τ($D_2O/NaHCO_3$), 2.6-3.15 (phenyl), 4.41 (d, J 5 Hz, $C_7$-H), 5.01 (d, J 5Hz, $C_6$-H), 5.2 (ABq, J 14 Hz, $C_3$-$C\underline{H}_2$-), 5.21 ($PhOC\underline{H}_2$-), 6.0-6.35 (C$\underline{H}$-NH and $-CO_2C\underline{H}_2$-), 6.62 and 6.93 (ABq, J 18 Hz, 2H: $C_2$-H), 7.65 ($-C\underline{H}_2CONH-$), 8.0-8.5 [$-C\underline{H}(CH_3)_2$ and 4H: CH-$C\underline{H}_2$-$C\underline{H}_2$-] and 9.1 [S, J 7 Hz, $(CH_3)_2C$]; λmax's (pH 6 phosphate) at 339.5 (ε 820) and 263 nm (ε 7995) and λinf's at 258.5 (ε 7745), 266.5 (ε 7995) and 274 nm (ε 6610); sodium content 3.3%, $C_{27}H_{32}O_{11}SN_3Na$ requires 3.6%.

<u>Example 7</u>

<u>(6R,7R)-7-[D-5-Benzamido-5-carboxypentanamido]-3-(1-pyridiniummethyl)ceph-3-em-4-carboxylate</u>

The product of Example 1 (5g) was dissolved in water (14ml) containing sodium hydrogen carbonate (0.66g). Pyridine (2.92ml) and potassium thiocyanate (8.8g) were added and the mixture was stirred at 55-60° for 40 minutes. After pouring the warm mixture into acetone (800ml), the solid which deposited was collected by vacuum filtration, washed with acetone then diethyl ether and sucked down briefly.

The wet cake was dissolved in water (70ml), and formic acid (2.86ml) was added before the mixture was stirred with Amberlite LA2 resin (32ml) in diethyl ether (80ml) for <u>ca</u> 5 minutes. The organic layer was backwashed with water (16ml) and the combined aqueous layers were extracted with diethyl ether before concentrating by rotary evaporation to low bulk (13.6g). Dropwise addition of orthophosphoric acid lowered the pH from 5.3 to 2.6 and after ageing for 90 minutes in the cold, the crystalline product was collected by vacuum filtration, washed with chilled water, then acetone and dried <u>in vacuo</u> at room temperature overnight to give the <u>title compound</u> (2.25g). UV (pH 6.0 buffer)

$E^{1\%}_{1cm}$ 285 at $\lambda_{max}$ 236.5 and $E^{1\%}_{1cm}$ 283 at $\lambda_{max}$ 253.5; τ (TFA) had inter alia 0.98 (d,J 6Hz, α-pyridinium) 1.35 (t J 6Hz, γ-pyridinium), 1.95 (t, J 6Hz, β-pyridinium), 3.80 and 4.63 (ABq, J 16Hz, $-CH_2$-pyr.) 6.18 and 6.65 (ABq, J 18Hz, $S-CH_2-$).

The title compound is described and claimed in European Patent Application Specification No. 70706, and as described in that Specification it may be used to prepare (6R,7R)-7-amino-3-(1-pyridiniummethyl)ceph-3-em-4-carboxylate dihydrochloride, for subsequent conversion into antibacterially active 7-acylamido-3-pyridiniummethyl cephalosporins.

EXAMPLE 8

(6R,7R)-7-[(Z)-2-(Fur-2-yl)-2-methoxyiminoacetamido]-3-(phenoxyacetoxymethyl)ceph-3-em-4-carboxylic acid

(6R,7R)-3-(Hydroxymethyl)-7-[(Z)-2-(fur-2-yl)-2-methoxyiminoacetamido]ceph-3-em-4-carboxylic acid (30g) was dissolved in N,N-dimethylacetamide (180ml) at 0° with stirring and treated dropwise with phenoxyacetyl chloride (24ml). The mixture was allowed to warm to ambient temperature over 3h before being partitioned between water (160ml) and ethyl acetate (300ml). The organic layer was washed successively with water, saturated brine and more water and then evaporated to dryness under reduced pressure. The residual oil was dissolved in ethyl acetate (50ml) and treated with a solution of sodium 2-ethylhexanoate (13g) in ethyl acetate (50ml). The resultant solution was evaporated to a small volume under reduced pressure. The oily product was mixed with ether (ca 1 l) to give a solid. The solid was filtered, washed well with ether and dried in vacuo at 40° to afford the title compound as its sodium salt (43.4g).

The sodium salt (10g) was dissolved in water (150ml) by the addition of sodium bicarbonate solution

to pH 6 and the clear solution was extracted with ethyl acetate (150ml). Fresh ethyl acetate was added to the aqueous layer and the mixture acidified to pH 2 with phosphoric acid. The layers were separated and the organic layer washed with water and evaporated to low volume. The oily product was treated with ether (100ml) and stirred for 10 min. before light petroleum (b.p. 60-80°) (100ml) was added to precipitate a solid. The solid was collected by filtration, washed well with light petroleum (b.p. 60-80°) then dried at 40° <u>in vacuo</u> to give the <u>title compound</u> as a powder (4.5g). τ (DMSO-$d_6$) 0.32 (d, J9Hz, $C_7$-NH), 2.21, 3.32 and 3.42 (multiplets, furyl protons), 2.6-3.2 (m, phenyl protons), 4.22 (dd, J9 Hz and 5 Hz, $C_7$-proton), 4.90 and 5.22 (ABq, J13 Hz, $C_3$-$C\underline{H}_2$), 5.20 (s, .$C\underline{H}_2$.OPh), and 6.10 (s, - $OC\underline{H}_3$); $\nu_{max}$ (Nujol) 3400-2500 (bonded -OH), 3285 (-NH), 1787 (β-lactam), 1763 (-$OCOCH_2$-), 1738 (-$CO_2H$), 1686 and 1534 (-CONH)$cm^{-1}$.

<u>EXAMPLE 9</u>

<u>Sodium (6R,7R)-3-phenoxyacetoxymethyl-7-[(Z)-2-(2-phenoxyacetamidothiazol-4-yl)-2-(2-carboxyprop-2-oxyimino)-acetamido]ceph-3-em-4-carboxylate</u>

Sodium (6R,7R)-3-(hydroxymethyl)-7-[(Z)-2-(2-aminothiazol-4-yl)-2-(2-carboxyprop-2-oxyimino)acetamido]ceph-3-em-4-carboxylate (4.6g) was suspended in N,N-dimethylacetamide (50ml) at 0° with stirring, and phenoxyacetyl chloride (7.4ml) was added in one charge. After stirring the mixture for 30 minutes at 0°, t.l.c. (silica/$CHCl_3$:methanol:$CH_3CO_2H$, 9:2:1) showed complete conversion of the starting material into a product with Rf 0.20. Following an extractive work-up, the <u>title compound</u> was isolated as a sodium salt (450mg). Rf 0.20 (silica/$CHCl_3$:methanol:$CH_3CO_2H$, 9:2:1); τ(DMSO-$d_6$) 0.18 (d, J9 Hz, $C_7$-NH), 2.6-3.2 (m, phenyl protons), 3.28

(s, aminothiazole proton), 4.9-5.6 (multiplets, acetyl protons and $C_2$-protons), and 8.51 (s, $C(C\underline{H}_3)_2$); $\nu_{max}$ (Nujol) 3700-3100 (NH and $H_2O$), 1767 (β-lactam), 1679 (CONH), 1536 (CONH), 1602 ($CO_2^{\ominus}$)$cm^{-1}$.

CLAIMS

1. A cephalosporin compound having a phenoxyacetoxymethyl group at the 3-position.

2. A cephalosporin compound of general formula

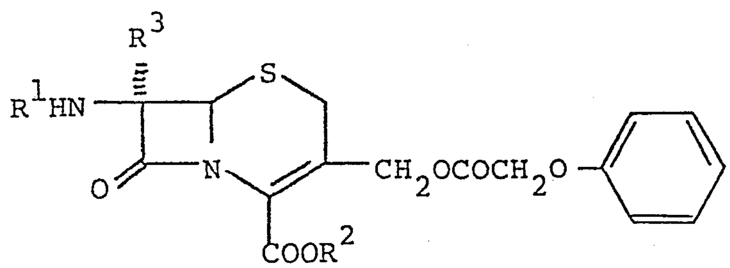

(I)

(wherein $R^1$ represents a hydrogen atom or an acyl group; $R^2$ represents a hydrogen atom or a carboxyl blocking group; and $R^3$ represents a hydrogen atom or a lower alkyl, alkylthio or alkoxy group) and, where appropriate, salts thereof.

3. A compound according to claim 2 wherein $R^2$ is a hydrogen atom.

4. A compound according to either of claims 2 and 3 wherein $R^1$ is a group of formula 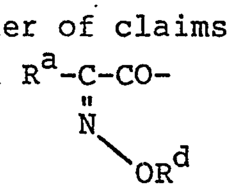

[wherein $R^a$ is an optionally substituted aryl (carbocyclic or heterocyclic), cycloalkyl, cycloalkadienyl or non-aromatic or mesoionic heterocyclic group; $R^d$ is a hydrogen atom, an optionally substituted lower alkyl or cycloalkyl group, or an acyl group; and the group $-OR^d$ is in the *syn*-configuration with respect to the carbonyl group.

5. A compound according to claim 2 which is

(III)

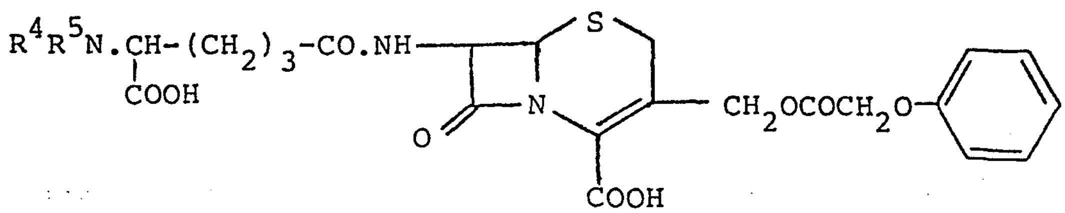

(wherein $R^4$ is hydrogen and $R^5$ is a monovalent amino protecting group, or $R^4$ and $R^5$ together form a divalent amino protecting group) and salts thereof.

6. A process for the preparation of a compound as claimed in claim 1 in which a cephalosporin compound having a hydroxymethyl group at the 3-position is subjected to phenoxyacetylation.

7. A process for the preparation of a compound according to claim 2 which comprises phenoxyacetylating a compound of formula

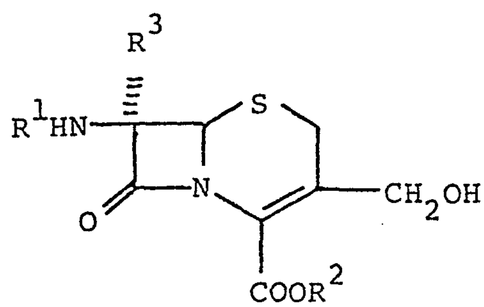

(II)

(wherein $R^1$, $R^2$ and $R^3$ are as defined in claim 2) or, where appropriate, a salt thereof.

8. A process according to claim 7 wherein any of the following reactions in any appropriate sequence, are carried out after the phenoxyacetylation:-

(i) N-deacylation of a compound wherein $R^1$ is an acylamido group to form a 7-amino compound;

(ii) N-acylation of a compound wherein $R^1$ is hydrogen to form a desired acylamido group;

(iii) conversion of a carboxyl group into a salt or ester function;

(iv) removal of any carboxyl blocking group, O-protecting, S-protecting and/or N-protecting groups; and

(v) conversion of a basic group into a salt function.

9. A process for the preparation of a cephalosporin compound having a 3-(substituted methyl) group which comprises subjecting a compound according to claim 1 to a nucleophilic displacement reaction to form the desired 3-substituent.

10. A pharmaceutical composition comprising an antibiotic compound according to any one of claims 1-4 in association with a pharmaceutical carrier or excipient.